# EUROPÄISCHE PATENTSCHRIFT

(11) **EP 0 568 676 B1**
(45) Veröffentlichungstag und Bekanntmachung des Hinweises auf die Patenterteilung: **17.01.1996**
(21) Anmeldenummer: 92923803.8
(22) Anmeldetag: 26.11.1992
(51) Int. Cl.: A61F 2/28

(54) **ENDOPROTHESE**
ENDOPROSTHESIS
ENDOPROTHESE

(30) Priorität: 02.12.1991 DE 9114970 U
(43) Veröffentlichungstag der Anmeldung: 10.11.1993
(73) Patentinhaber: Waldemar Link (GmbH & Co.), D-22315 Hamburg (DE)
(72) Erfinder: KELLER, Arnold, D-2061 Kayhude (DE)
(74) Vertreter: Glawe, Delfs, Moll & Partner
(86) Internationale Anmeldenummer: EP9202730
(87) Internationale Veröffentlichungsnummer: WO9310724

(56) Entgegenhaltungen:
- US-A- 4 578 081

## Beschreibung

Die Erfindung betrifft eine Endoprothese mit zwei durch Überlappungsverbindung verbundenen Prothesenteilen.

Es besteht häufig das Problem, zwei Prothesenteile nach deren gesonderter Implantation starr miteinander zu verbinden. Ein Beispiel dafür ist eine Prothese zur starren Verbindung zweier Knochenfragmente oder zur Arthrodese zweier sonst durch Gelenk verbundener Röhrenknochen. An der Bruch- bzw. Gelenkstelle können die beiden Knochen gegeneinander abgewinkelt werden, so daß die Schäfte der beiden Prothesen in die Knochen eingeführt werden können. Danach werden die Knochen zueinander ausgerichtet und die einander zugewendeten Teile der Prothesen miteinander verbunden. Dies kann bspw. formschlüssig unter Verwendung eines Konus oder Zapfens am Ende des einen Schafts geschehen, der in eine entsprechende Hülse am Ende des anderen Schafts eingeführt wird. Dies hat aber den Nachteil, daß zum Einfügen des Kegels oder Zapfens in die zugehörige Hülse die beiden Knochen zunächst um die Kegel- bzw. Zapfenlänge distrahiert werden müssen, was schwierig oder schädlich sein kann. Erwünscht ist deshalb eine Verbindung, die ohne wesentliche Distraktion geschlossen werden kann. Dafür steht die Laschenverbindung zur Verfügung, bei der zwei an den Enden der Prothesenschäfte vorgesehenen Laschen miteinander verschraubt werden, was jedoch den Nachteil hat, daß die Verläßlichkeit der Verbindung von den Schrauben abhängt, die sich lockern können, und daß der Übergang von den Laschen zu den Schäften eine Schwachstelle bildet.

Dokument US-A-4578081 beschreibt eine Endoprothese gemäß dem Oberbegriff des Anspruchs 1.

Die Erfindung vermeidet diese Nachteile bei einer Laschenverbindung dadurch, daß die Enden der einander überlappenden Laschen formschlüssig von Taschen am jeweils anderen Prothesenteil aufgenommen sind. Um die Laschen miteinander zu kuppeln, ist lediglich eine geringe Distraktion erforderlich, deren Länge der Länge des Eingriffs der Laschenenden in die zugehörigen Taschen entspricht. Diese Länge ist wesentlich geringer als bei einer Zapfen- bzw. Kegel-Verbindung, bei der eine Distraktion über praktisch die gesamte Länge des Verbindungsbereichs erforderlich ist.

Erfindungsgemäß genügt eine Eingriffslänge der Laschenenden in die zugehörigen Taschen von nicht mehr als 1/5, vorzugsweise nicht mehr als 1/10 der gesamten Überlappungslänge.

Zweckmäßigerweise sind Einrichtungen zur Sicherung der Laschen in der Eingriffsstellung vorgesehen, die lediglich dafür zu sorgen brauchen, daß keine Längsverschiebung der Laschen zueinander stattfinden kann, durch welche die Laschenenden aus den zugehörigen Taschen herausgelangen könnten. Die einschlägige Technik stellt derartige Mittel in verschiedener Form zur Verfügung. Am einfachsten ist es, die Laschen miteinander zu verschrauben. Bemerkenswert ist dabei, daß die Verschraubung die Längsverschiebung auch dann verhindert, wenn sich eine Schraube ein wenig gelockert haben sollte. Im Unterschied zu den bekannten, verschraubten Laschenverbindungen wird daher die Sicherheit der Verbindung durch die Schraubenlockerung nicht beeinträchtigt.

Nach einem weiteren Merkmal der Erfindung sind die Laschen nahe ihrem Übergang in den zugehörigen Prothesenteil dicker als an ihrem freien Ende, indem die Anlagefläche, die sie einander zukehren, geneigt zur gemeinsamen Längsachse des Verbindungsbereichs der Prothesenteile verläuft. Dadurch daß die Laschen an demjenigen Ende, an welchem sie mit dem zugehörigen Prothesenteil fest verbunden sind, am dicksten sind, können sie querschnittsgleich ohne Ausbildung gefährlicher Kerben in den Prothesenteil übergehen, während ihr in die Tasche des anderen Prothesenteil eingreifende Ende entsprechend dünner ist. Die Schrägung erlaubt es, die Laschenenden und die sie umfassenden Taschen ineinander zu verkeilen, so daß sich eine selbsthemmende, spielfreie Eingriffsstellung ergibt.

Die Erfindung wird im folgenden näher unter Bezugnahme auf die Zeichnung erläutert, die ein vorteilhaftes Ausführungsbeispiel einer Prothese oder Schiene für die Arthrodese des Kniegelenks zeigt. Darin stellen dar:
- Fig. 1: den Verbindungsbereich der Prothese in Seitenansicht,
- Fig. 2: eine Seitenansicht quer zu derjenigen gemäß Fig. 1,
- Fig. 3 und 4: gesonderte Seitenansichten der Verbindungsbereiche der beiden Prothesenteile und
- Fig. 5 und 6: Längsansichten der Verbindungsbereiche der Prothesenteile.

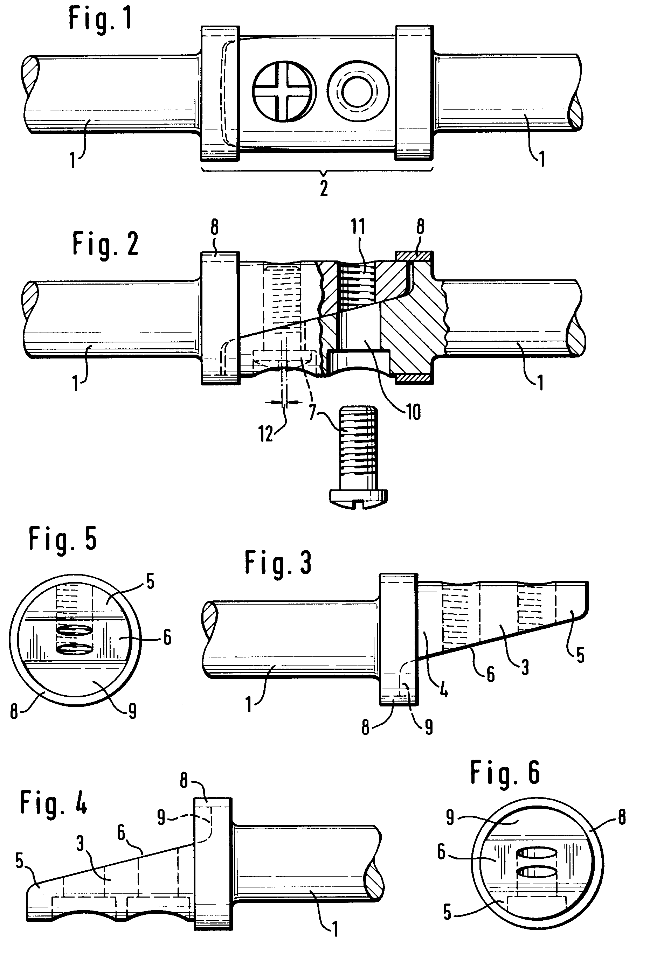

Die beiden Prothesenteile weisen je einen Schaft 1 zur Implantation im Ober- bzw. Unterschenkelknochen auf. Ihr Verbindungsbereich 2 umfaßt zwei einander zu zylindrischer Außenform ergänzende Laschen 3, deren Anlagefläche 6 so geneigt verläuft, daß jeweils das dickere Ende 4 einstückig und mit unverminderter Biegefestigkeit in den zugehörigen Schaftteil 1 übergeht, während das freie Ende 5 in der Darstellungsebene der Fig. 3 und 4 etwa halb so dick wie das dickere Ende 4 ist und seine Querschnittsfläche etwa 1/3 so groß ist. Die Laschen liegen längs der Fläche 6 im Verbindungszustand gemäß Fig. 1 und 2 flächig aneinander. Mittels Schrauben 7 können sie in dieser Stellung gesichert werden.

An der Wurzel jeder Lasche 3 ist diese von einem Ring 8 umschlossen, der eine Tasche 9 mit dem zugehörigen Ende der Anlagefläche 6 einschließt, deren Querschnitt in Längsansicht gemäß Fig. 5 und 6 demjenigen des Endteils 5 der jeweils anderen Lasche entspricht. Dabei sind die Abmessungen so gewählt, daß im spielfrei zusammengeschobenen Zustand, der in Fig. 3 dargestellt ist, die Anlageflächen 6 und die Umfangsflächen der Laschenenden 5 spielfrei miteinander bzw. dem jeweiligen Ring 8 in Kontakt kommen, so daß schon allein durch das Zusammenwirken der Laschenenden 5 mit den Taschen 9 eine starre Verbindung gewährleistet ist. Die Verbindungssicherheit wird dabei durch die Keilwirkung der Anlagefläche 6 unter der Prothesenbelastung, die die beiden Prothesenteile zusammenzuschieben versucht, noch gesteigert. In der Eingriffsstellung werden die Laschen durch Schrauben 7 gesichert, wobei die zusammengehörigen, gegenüberliegenden Schraublöcher 10, 11 so angeordnet sind, daß selbst dann, wenn sie im montierten Zustand ein wenig gegeneinander versetzt sind, wie dies bei 12 angedeutet ist, noch der vollständige Eingriff der Laschenenden 5 in den Taschen 9 gewährleistet ist. Der Ring 8 ist vorzugsweise fest an dem zugehörigen Prothesenteil befestigt.

Bei Verwendung von Schrauben zur Sicherung des Eingriffs können diese zur Kraftübertragung herangezogen und entsprechend dimensioniert werden.

## Patentansprüche

1. Endoprothese mit zwei durch einander überlappende Laschen verbundenen Prothesenteilen, dadurch gekennzeichnet, daß die Enden (5) der Laschen (3) formschlüssig von Taschen (9) am jeweils anderen Prothesenteil aufgenommen sind.

2. Endoprothese nach Anspruch 1, dadurch gekennzeichnet, daß die Taschen (9) eine Länge von nicht mehr als 1/5, vorzugsweise nicht mehr als 1/10, der gesamten Laschenlänge aufweisen.

3. Endoprothese nach Anspruch 1 oder 2, dadurch gekennzeichnet, daß die Laschen (3) mit Einrichtungen zur Sicherung in der Eingriffsstellung ausgerüstet sind.

4. Endoprothese nach Anspruch 3, dadurch gekennzeichnet, daß die Einrichtungen zur Sicherung in der Eingriffsstellung von mindestens einer Schraube (7) gebildet sind.

5. Endoprothese nach einem der Ansprüche 1 bis 4, dadurch gekennzeichnet, daß die Laschen (3) nahe ihrem Übergang in den zugehörigen Prothesenteil dicker als an ihrem freien Ende sind.

6. Endoprothese nach Anspruch 5, dadurch gekennzeichnet, daß die Anlagefläche (6), die die Laschen (3) einander zukehren, geneigt zur Längsachse des Verbindungsbereichs (2) der Prothese verläuft.

7. Endoprothese nach Anspruch 5 oder 6, dadurch gekennzeichnet, daß die Enden (5) der Überlappungsteile (3) und die Taschen (9) in der Eingriffsstellung spielfrei aufgrund Keilwirkung zusammenwirken.

## Claims

1. An endoprosthesis with two prosthesis parts connected by mutually overlapping plates, characterised in that the ends (5) of the plates (3) are accommodated in a form-locking manner by sockets (9) in the respective other prosthesis part.

2. An endoprosthesis according to Claim 1, characterised in that the sockets (9) are of a length of not more than 1/5, preferably not more than 1/10, of the entire plate length.

3. An endoprosthesis according to Claim 1 or 2, characterised in that the plates (3) are provided with means for securing in the engagement position.

4. An endoprosthesis according to Claim 3, characterised in that the means for securing in the engagement position are formed by at least one screw (7).

5. An endoprosthesis according to any one of Claims 1 to 4, characterised in that near to their transition into the associated prosthesis part the plates (3) are thicker than at their free end.

6. An endoprosthesis according to Claim 5, characterised in that the abutment surface (6), with which the plates (3) face one another, extends inclined relative to the longitudinal axis of the connecting zone (2) of the prosthesis.

7. An endoprosthesis according to Claim 5 or 6, characterised in that the ends of the overlapping parts (3) and the sockets (9) co-operate in the engaged position without play as a result of a wedging action.

## Revendications

1. Endoprothèse en deux parties jointes par des tenons se chevauchant, caractérisée en ce que les extrémités (5) des tenons (3) sont reçues à ajustement étroit dans des poches (9) sur la partie de prothèse respectivement opposée.

2. Endoprothèse selon la revendication 1, caractérisée en ce que les poches (9) ont une longueur qui ne dépasse pas 1/5 et, de préférence, ne dépasse pas 1/10 de la longueur totale du tenon.

3. Endoprothèse selon la revendication 1 ou 2, caractérisée en ce que les tenons (3) sont munis de dispositifs pour les assujettir dans leur position d'engagement.

4. Endoprothèse selon la revendication 4, caractérisée en ce que les dispositifs pour l'assujettissement en position engagée sont constitués par au moins une vis (7).

5. Endoprothèse selon l'une quelconque des revendications 1 à 4, caractérisée en ce qu'à proximité de leur jonction à la partie de prothèse dont ils font partie, les tenons sont plus épais qu'à leur extrémité libre.

6. Endoprothèse selon la revendication 5, caractérisée en ce que la surface de contact (6) que les tenons (3) opposent l'un à l'autre est inclinée par rapport à l'axe longitudinal de la région d'assemblage (2) de la prothèse.

7. Endoprothèse selon la revendication 5 ou 6, caractérisée en ce qu'en position engagée, les extrémités (5) des parties chevauchantes (3) et les poches (9) coopèrent sans jeu en conséquence de l'effet de coin.
